# EUROPEAN PATENT APPLICATION

(11) **EP 4 819 153 A1**
(43) Date of publication of application: **07.10.2026**
(21) Application number: 25382326.4
(22) Date of filing: 01.04.2025
(51) Int. Cl.: G16H 30/40, A61B 5/026, G16H 50/20, G16H 50/50

(54) **METHOD AND APPARATUS FOR SIMULATING ABDOMINAL HEMODYNAMIC BEHAVIOUR**

(71) Applicant: Hospital Clínic de Barcelona, 08036 Barcelona (ES); Fundació de Recerca Clínic Barcelona-Institut d'Investigacions Biomèdiques August Pi I Sunyer, 08036 Barcelona (ES)
(72) Inventor: AUSANIA, Fabio, 08036 Barcelona (ES); GONZÁLEZ ABÓS, Carolina, 08036 Barcelona (ES)
(74) Representative: TRBL Intellectual Property

(57) **Abstract**

The present invention relates to a method for predicting post-surgical or post-treatment blood flow redistribution in a subject's abdominal vascular system that takes into account the full complexity of said abdominal vascular system and the interplay between different compensation biological mechanisms in the abdominal blood flow. The proposed method comprises providing a 3D model of the vascular system of an abdominal region of the subject based on medical images, generating a computational model of blood flow through the subject's abdominal vascular system based on the 3D model, receiving blood-flow modification conditions at a location of the subject's abdominal vascular system and updating the computation model to include the blood-flow modification conditions inputted. The method includes the computational segmentation and reconstruction of at least the common hepatic artery, the gastroduodenal artery, the celiac trunk, and its arcades, the proper hepatic artery, the superior mesenteric artery and the splenic artery as well as means for applying the hepatic artery buffer response when necessary. Associated apparatuses and computer programs are also disclosed.

## Description

### FIELD OF THE INVENTION

The present invention belongs to the technical field of medical computational simulation for preoperative and/or treatment planning. More specifically, the invention relates to a computational method for predicting post-surgical or post-treatment blood flow redistribution in a subject's abdominal vascular system that takes into account the full complexity of said abdominal vascular system and the interplay between different compensation biological mechanisms in the abdominal blood flow. Associated apparatuses and computer programs are also disclosed.

### BACKGROUND OF THE INVENTION

Major hepatobiliary and pancreatic (HPB) surgeries are mostly complex procedures associated with high morbidity. An accurate preoperative planning is essential for allowing a safe surgery in these patients, specially relating to knowledge of arterial anatomy and its variations, which is a crucial prerequisite to perform safe HPB surgery. Only approximately 55% of patients show a normal anatomy according to Michels classification, which may lead to post-surgical complications if blood flow redistribution after HPB surgery is not as expected.

Blood supply to abdominal organs mainly originates from the celiac trunk (CT) and superior mesenteric artery (SMA). Pancreatic surgery requires the interruption of some of the arteries arising from these vessels and pancreaticoduodenectomy (PD) implies gastroduodenal (GDA) and inferior pancreaticoduodenal (IPDA) arterial ligation. These latter arteries represent the most important communication between CT and SMA arterial systems: an arterial insufficiency of one system can be counterbalanced by hypertrophy of the other one. For this reason, many significant CT stenoses can be undiagnosed before surgery. In fact, up to 10% of patients undergoing PD show a CT stenosis, and in these patients GDA ligation can lead to increased major complications, including biliary and liver ischemia.

The use of last generation multidetector computer tomography (MDCT) allows high-quality assessment of vascular anatomy and its variations such as arterial diseases like stenosis and/or arteriosclerosis, which can lead to altered blood supply. However, although preoperative MDCT can detect, for example, a significant CT stenosis with high sensitivity, the hemodynamic impact of the stenosis is difficult to predict.

Doppler ultrasound can contribute to a better assessment, allowing the measurement of CT and common hepatic artery (CHA) blood flow, although its feasibility is limited by patient characteristics such as obesity, anatomical variability and the difficulty of assessing smaller calibre arteries such as the GDA.

Four-dimensional (4D) flow magnetic resonance allows comprehensive in vivo measurement of three-dimensional blood flow dynamics in large vessels, but its accuracy in small and tortuous vessels, such as the CHA, is reduced due to blood turbulence. Besides that, in some exceptional cases, CT resection is needed for treatment of a pancreatic body neoplasm that affects the CT, and in these cases prediction of future blood flow from the GDA to the liver is needed. However, none of the previous technologies provides any information on the effect of GDA ligation.

Arteriography with hepatic artery occlusion is the only test that can be helpful to predict the hemodynamic effect of GDA ligation or CHA ligation but it is an invasive test with a non-negligible risk of complications. Other technologies, such as dynamic contrast enhanced CT or PET/CT, have been used to assess organic perfusion in the brain, but have not been studied in the abdomen due to its complex anatomy.

Computational fluid dynamics (CFD) has previously been successful in revealing distinct features of hemodynamic disturbances in cardiovascular diseases such as aortic aneurysms and dissections. CFD enables the study of various conditions that may not be present in MDCT current images by modifying the geometry of the vascular system model in which it is based or deployed. This flexibility allows for the simulation and analysis of hypothetical scenarios and the impact of different pathological changes on blood flow dynamics.

Currently, in the evaluation of coronary arteries, computational flow analysis by CT has shown a higher performance for the diagnosis of significant vascular stenosis in patients with coronary artery disease, thus presenting CFD as a good alternative in the noninvasive evaluation of blood flow. CFD has also been used to analyze flow distribution in hepatic artery branches and portal blood flow redistribution after major hepatectomy, but these studies have only analyzed a small number of cases. While noninvasive assessment of blood flow has become a well-established practice in cardiovascular disease, its application in the abdomen is not yet sufficiently developed.

This discrepancy arises from several challenges including the complex anatomy and diverse blood flow patterns within the abdominal cavity, involving multiple vessels supplying various organs and presenting considerable interindividual variability. According to the current literature discussing CFD, this technology seems to be an interesting tool to study the blood flow of the supramesocolic compartment in in silico models, but some issues should be considered: 1) the non-Newtonian behavior of blood may alter the primary and secondary flow patterns and 2) it is not possible to fully capture how boundary conditions influence detailed flow characteristics, such as velocity fields, flow lines, and particle trajectories. In addition, CFD-generated blood flow simulations do not perfectly replicate in vivo conditions.

For all these reasons, to date, a computational model capable of comprehensively studying the supramesocolic compartment of the human body and assessing how different surgical maneuvers affect the redistribution of vascular flow in this region is yet to be developed. Most existing studies tend to focus on specific organs or systems within the abdomen, without considering the complex hemodynamic interactions that can occur when performing surgical procedures, especially when they affect the vessels interconnecting the CT and SMA system. This lack of integrated models limits the ability of surgeons to predict the side effects of interventions that modify blood circulation, such as the resection of some organs, or the creation of a vascular anastomosis.

However, predicting blood supply to abdominal organs is of paramount importance, especially in major HPB surgery, because hepatic artery insufficiency caused by GDA ligation is associated with clinically relevant pancreatic fistula, liver perfusion failure, gastric ischemia, clinically relevant biliary leakage and the need for intensive care. The presence of arterial disease in patients undergoing surgery is a current concern as more elderly patients are considered for aggressive surgery and the presence of metabolic syndrome is increasing in the general population. Although the presence of arterial calcifications or stenosis at preoperative MDCT is common, their hemodynamic impact is unknown, and it is difficult to predict without an invasive test such as arteriography.

In view of the above, there is a need in the field of a non-invasive method for predicting post-surgical blood flow redistribution in a subject's abdominal vascular system in patients undergoing hepatobiliary and pancreatic surgery that takes into account the full complexity of said abdominal vascular system and the interplay between different compensation biological mechanisms in the abdominal blood flow.

### BRIEF DESCRIPTION OF THE INVENTION

The present invention overcomes the above limitations of the prior art, by providing a computational method that, based on medical imaging of a subject's abdominal region (such as MDCT images), is adapted to predict the redistribution of the blood flow in the subject's abdominal vascular system after a modification has taken place in said vascular system, as it occurs in hepatobiliary and pancreatic surgeries. The method of the invention is further adapted to accurately model the main blood vessels in the abdominal cavity, i.e., the CT, the SMA, the CHA, the GDA, the CT and its arcades, the proper hepatic artery (PHA), the splenic artery (SA) and the IPDA as well as to implement the biological compensatory mechanisms between them. For instance, the predictions obtained from the method of the invention take into account the hepatic artery buffer response (HABR), which ensure sufficient hepatic perfusion, that is related to the interplay between the CT and SMA blood flows and to a decrease in the portal flow.

Hence, the present invention relates in a first aspect to a computer-implemented method for predicting post-surgical blood flow redistribution in a subject's abdominal vascular system, by performing the following steps:
a) providing a three-dimensional (3D) digital model of a vascular system of an abdominal region of the subject, based on one or more medical images of said abdominal region;
b) generating an initial computational model of blood flow through the subject's abdominal vascular system, based on the 3D digital model;
c) receiving input information indicating one or more blood-flow modification conditions at a location of the subject's abdominal vascular system;
d) updating the computational model to include the blood-flow modification conditions inputted in step c); and
e) outputting a representation of the redistribution of the abdominal blood flow of the subject after the abdominal vascular system modification/s inputted in step c) according to the model updated in step d).

Advantageously and specifically designed for accurately predicting the hemodynamic behaviour in the abdominal region of a subject, the method is characterized in that:
- the 3D model built in step a) comprises the computational segmentation of at least the common hepatic artery, the gastroduodenal artery, the celiac trunk and its arcades, the proper hepatic artery, the superior mesenteric artery, the splenic artery, and the inferior pancreaticoduodenal artery within the one or more medical images of the abdominal region of the subject, and the posterior reconstruction of said vessels' geometries;
- the computational model is adapted to apply a biological mechanism of blood flow compensation between the CT and the SMA, by increasing, in a predefined amount, the modelled CT/SMA blood flow if the 3D model provided in step a) or if one of the modifications inputted in step c) is related to a reduction in the normal SMA/CT blood flow; and in that
- the computational model updated in step d) is adapted to apply the hepatic artery buffer response, HABR, by increasing, in a predefined amount, the modelled PHA blood flow if one of the modifications inputted in step c) corresponds to the ligation or clamping of the GDA and/or to the ligation or clamping of the SA, secondary to a modification of portal vein (PV) blood flow..

The described method provides an accurate way of predicting blood flow redistribution in the abdominal region of a patient/subject as a response to an anatomical modification such that those that occur during medical treatments, especially in hepatobiliary and pancreatic surgeries. This is achieved thanks to its design specifically focused on the abdominal vascular system's anatomy and on the connections and interplay between the different vessels comprised therein.

In particular, the method is optimized so that it accounts for important effects of the abdominal vascular system such as the compensation mechanism between the CT and SMA blood flows, in which, when one decreases (for example in cases of CT stenosis) the other increases, and such as the HABR, triggered by the interaction between the CT and the SMA after GDA or CHA ligation or by the interaction between the PHA and the portal vein after SA ligation. These are peculiar and non-trivial characteristics of the supramesolic irrigation system that other methods in the art, although aimed at simulating or predicting blood flow redistribution, fail to include.

An accurate and reliable way of predicting hemodynamic behaviour after an anatomical modification in the abdominal vascular system is of outmost importance since postsurgical complications due to, for instance, insufficient hepatic perfusion, are relatively common but can be avoided with dedicated planning. Early recognition of vascular alterations is crucial, since, if detected during surgery, it may be necessary to suspend the procedure to avoid serious consequences for the patient. Preoperative detection of possible post-surgical problems by implementing the method of the invention allows to solve them before the surgery is performed, either endovascularly or with a different surgical planning that allows for their surgical resolution. This is only possible if all the described effects and interplay between the different structures of the abdominal vascular system are taken into account.

In particular embodiments of the invention, the computational model obtained in step b) and updated in step d) comprises an algorithm based on computational fluid dynamics. Preferably, said algorithm comprises a Windkessel model, and even more preferably, the algorithm is based on artificial intelligence (AI) techniques adapted to provide test data of the blood flow through a test subject's abdominal vascular system based on a 3D digital model of said vascular system using as input real or synthetic physiological training data of a plurality of training subjects. Optionally, the Windkessel model may comprise fixed parameter values except for a resistance parameter.

Computational fluid dynamics allows for obtaining accurate predictions of the behaviour of the blood through the abdominal vascular system once the geometry of said system has been modelled (3D model). The use of a Windkessel model accounts for the shape of arterial blood pressure waveform in terms of the interaction between the stroke volume and the compliance of large elastic arteries (Windkessel vessels) and the resistance of the smaller arteries and arterioles. It may be therefore applied with the two mentioned parameters as free quantities, adapting their values to the needs of the procedure, or it can be used fixing one parameter and leaving the resistance parameter free. This implies using constant flow values instead of pulsatile flow values, which reduces significantly the computational time of execution of the algorithm with little or no detriment to the results.

The use artificial intelligence (AI) techniques allows for building an initial model based and trained on already collected data from different patient's records. Once the model is built and validated, a new piece of data can be inputted to said model obtaining a blood flow prediction. This increases the accuracy of the method and makes it more robust the more training data used.

In particular embodiments of the invention, the algorithm comprises a set of parameters and the parameter's initial values are set using:
- data associated with intraoperative measurements of arterial and/or venous blood flows of the subject; and/or
- data inferred by means of executing an AI algorithm adapted to provide an estimate of an initial blood flow of one or more abdominal vessels using as input at least one of the following: data from blood flow intraoperative measurements of a plurality of subjects, subject's demographics, subject's clinical variables, and subject's imaging-derived features.

The main advantage of using data associated with intraoperative measurements of arterial and/or venous blood flows of the subject to adjust the parameter values is that the method of the invention increases in accuracy for that specific subject, since possible physiological variations are eliminated. On the other hand, the main advantage of using data obtained by means of an AI algorithm is that it can be completely safe and non-invasive for the subject, and can be sufficiently accurate if enough data is used for training. Also, by incorporating clinical patient variables along with features extracted by CT radiomics, the AI model could identify hidden patterns in the flow distribution that escape conventional analysis, providing more accurate estimates of initial flows, reducing reliance on costly techniques such as 4D flow MRI. In addition, it would allow flow values to be dynamically studied as a function of multiple patient parameters, and the change of these parameters would allow the patient's flow to be measured dynamically.

Advantageously, the data associated with intraoperative measurements and/or the data inferred by means of executing an AI algorithm comprise data relating to the abdominal blood flow during HABR of:
- one or more subjects with a healthy liver; and/or
- one or more subjects with hepatic cirrhosis requiring a liver transplant;
which allows for taking into account the HABR effect more reliably, since in the first case (healthy liver) the interplay between the CT and the SMA may be more relevant to characterize the HABR whereas for the second case (cirrhotic liver) the regulatory mechanisms between the PHA and the PV provide better information on the HABR quantification.

In particular embodiments of the invention, the one or more medical images of the abdominal region of said subject comprise a computed tomography scan and/or a multidetector computer tomography scan. These are non-invasive procedures that have demonstrated to be suitable for building accurate 3D models of abdominal vascular systems and even for preliminary estimating blood flows of some of the vessels contained therein.

In particular embodiments of the invention, at least one of the modifications inputted in step c) comprises one of the following: clamping or ligation of the CHA, clamping or ligation of the GDA and clamping or ligation of the SA. These are the main anatomical modifications carried out in the abdominal vascular system during hepatobiliary or pancreatic surgical procedures.

In particular embodiments of the invention, steps c)-e) are repeated iteratively; the information inputted each time that step c) is carried out is based on a blood flow modification condition of the subject occurring in real time; and the update of the model and the output of the representation of the redistribution of the abdominal blood flow of the subject according to each modification condition inputted in step c) are carried out in real time.

This allows for assessing hemodynamic behaviour of the abdominal vascular system in real time, as, for instance, during surgery, in order to prevent peri-surgical complications. Moreover, it opens the possibility of building a digital twin of a subject that reacts in real time to different modifications in their anatomy.

In a second aspect, the invention relates to an apparatus for predicting post-surgical blood flow redistribution in a subject's abdominal vascular system, said apparatus comprising computing means comprising in turn hardware and/or software means adapted to perform a method according to any one of the described embodiments.

The apparatus may further comprise:
- medical image acquisition means adapted to obtain one or more images of the abdominal region of a subject; and/or
- one or more medical probes adapted to measure intraoperatively the blood flow in a vessel.

Finally, in a third aspect the present invention relates to a computer program comprising instructions which, when the program is executed by computing means, cause the computing means to carry out a method according to any one of the described embodiments.

All the terms and embodiments described anywhere in this document are equally applicable to all aspects of the invention. It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice.

### DESCRIPTION OF THE DRAWINGS

The foregoing and other features and advantages will be more fully understood from the detailed description of the invention, as well as from examples referring to the attached figures, which are described in the following paragraphs, wherein:

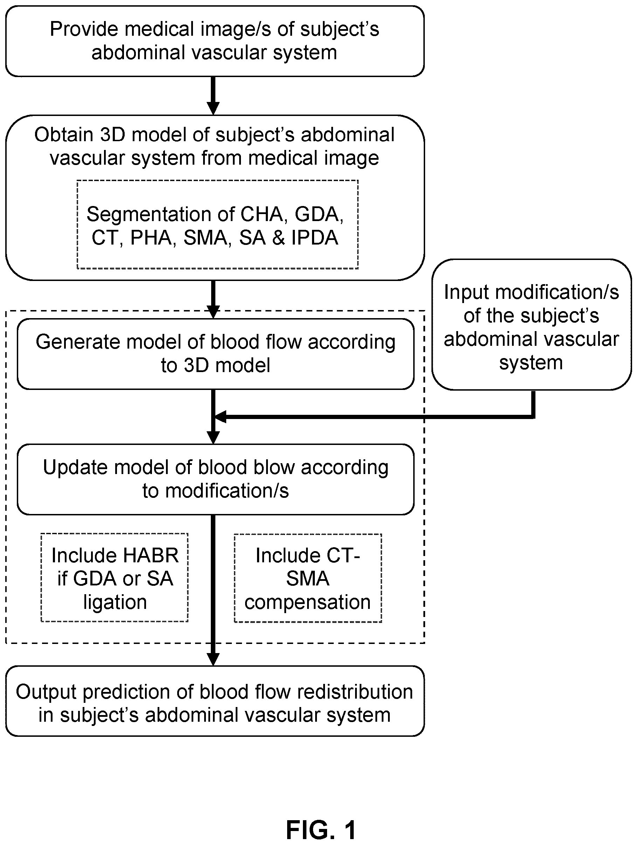

Figure 1 shows a schematic representation of the steps of a method according to an embodiment of the present invention.

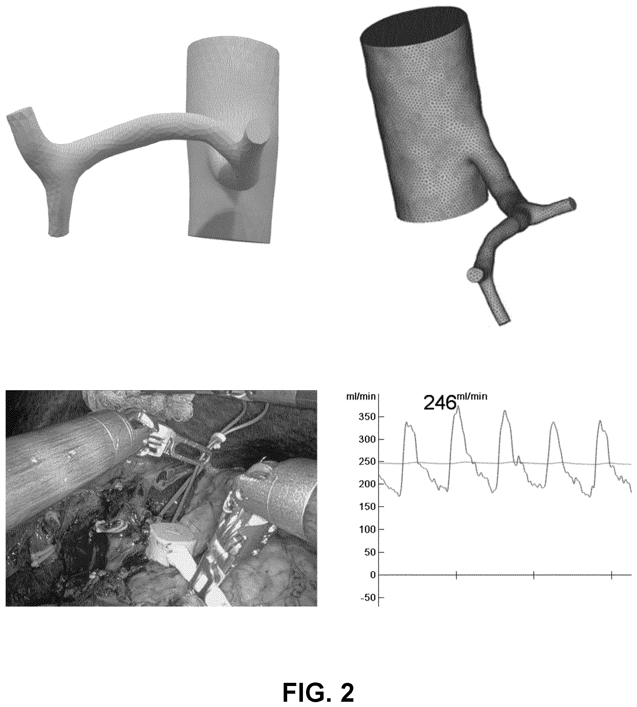

Figure 2 shows image segmentation, meshing of the segmented anatomy and intraoperative blood flow measurement according to an embodiment of the method of the present invention. Top left: segmentation of the CT, CHA, PHA and GDA starting from the aorta; top right: section of the mesh displaying distinct shades representing different labels, accompanied by visible perpendicular cuts at the exits; bottom left: intraoperative blood flow measurement of hepatic common artery during a robotic pancreaticoduodenectomy; bottom right: blood flow waveforms obtained from intraoperative measurement. An ACI>50% indicates good performance of the flow measurement.

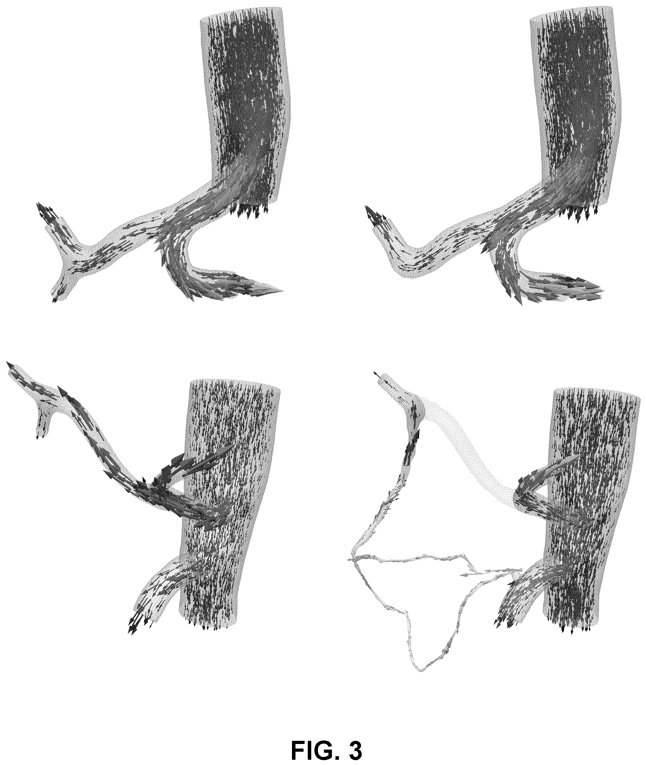

Figure 3 shows two computational fluid dynamics simulations (top panels and bottom panels) according to an embodiment of the method of the present invention. Top left: simulation of blood flow distribution in initial configuration of a first subject; top right: simulation of blood flow distribution after GDA clamping of the first subject; bottom left: simulation of blood flow distribution in initial configuration of a second subject; bottom right: simulation of blood flow redistribution after CHA clamping of the second subject.

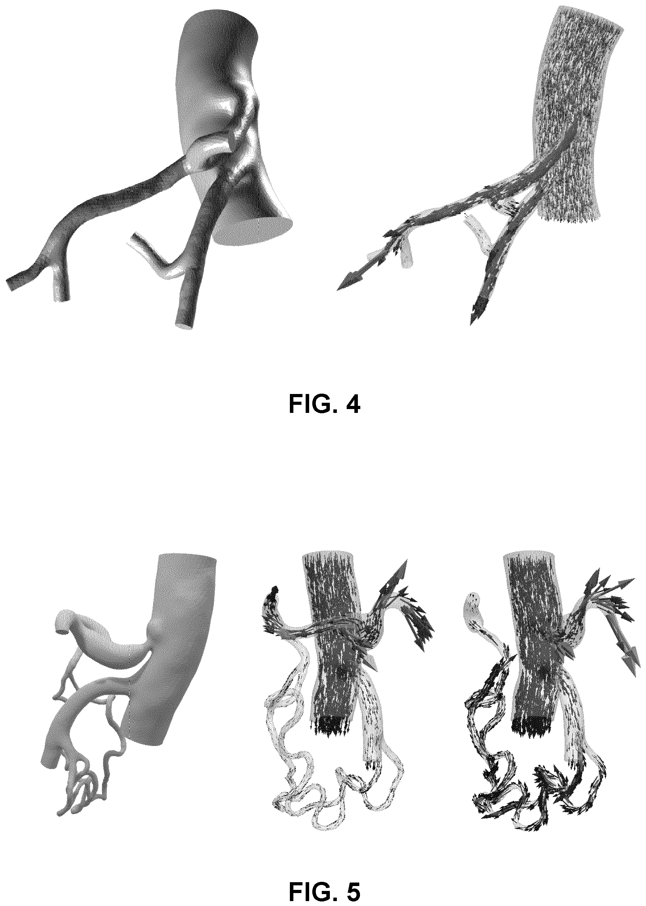

Figure 4 shows a blood flow simulation by performing the method of the invention for a subject with a replaced right hepatic artery from superior mesenteric artery. Left: model segmentation; right: predicted blood flow distribution. The right hepatic artery shows a blood flow of approximately 59 ml/min.
Figure 5 shows a blood flow simulation by performing the method of the invention for a subject with a clinically relevant celiac trunk stenosis. Left: model segmentation showing radiologically significant celiac trunk stenosis; middle: initial configuration, CHA and GDA are open; right: scenario 2, CHA is clamped. Approximately 150 ml/s are expected.

### DETAILED DESCRIPTION OF THE INVENTION

The following description refers to the accompanying drawings, which illustrate specific embodiments in which the present invention may be implemented. These embodiments will be described in detail sufficient to enable those skilled in the art to implement the present invention. It should be understood that various embodiments of the present invention are different from each other but need not be mutually exclusive. Accordingly, the detailed description to be described below is not intended to be taken in a limiting meaning, and the scope of the present invention, if properly described, is limited only by the appended claims, in addition to all scopes equivalent to those claimed by the appended claims. In the drawings, reference numerals refer to the same or similar functions over several aspects.

The present invention overcomes the above limitations of the prior art, by providing a computational method that, based on medical imaging of a subject's abdominal region, is adapted to predict the redistribution of the blood flow in the subject's abdominal vascular system after a modification has taken place in said vascular system, as it occurs in hepatobiliary and pancreatic surgeries. The method of the invention is further adapted to accurately model the main blood vessels in the abdominal cavity, as well as to implement the biological compensatory mechanisms between them.

Figure 1 depicts the main steps carried out in the performance of the method of the invention, according to a preferred embodiment. These steps allow for accurately and non-invasively simulating or predicting post-surgical blood flow redistribution in a subject's abdominal vascular system and correspond to:
a) providing a three-dimensional (3D) digital model of a vascular system of an abdominal region of the subject, based on one or more medical images of said abdominal region such as MDCT images;
b) generating an initial computational model of blood flow through the subject's abdominal vascular system, based on the 3D digital model, preferably using CFD;
c) receiving input information indicating one or more blood-flow modification conditions at a location of the subject's abdominal vascular system, mainly those performed during major hepatobiliary and pancreatic surgery, such as clamping or ligation of the CHA, GDA or SA;
d) updating the computational model to include the blood-flow modification conditions inputted in step c); and
e) outputting a representation of the redistribution of the abdominal blood flow of the subject after the abdominal vascular system modification/s inputted in step c) according to the model updated in step d).

Advantageously and specifically designed for accurately predicting the hemodynamic behaviour in the abdominal region of a subject, both the 3D model and the computational model comprise different features aimed at accounting for the peculiarities of the abdominal vascular system.

The 3D model comprises the computational segmentation of at least the common hepatic artery (CHA), the gastroduodenal artery (GDA), the celiac trunk (CT) and its arcades, the proper hepatic artery (PHA), the superior mesenteric artery (SMA), the splenic artery (SA), and the inferior pancreaticoduodenal artery (IPDA) within the one or more medical images of the abdominal region of the subject, and the posterior reconstruction of said vessels' geometries.

The computational model is adapted to apply different biological mechanisms that are crucial for obtaining a reliable prediction of the blood flow distribution in the abdominal region. The first of these mechanisms is that of blood flow compensation between the CT and the SMA. These arteries are connected through different vessels that allow to regulate their overall flow. In this way, when the blood flow of the CT decreases, the subject's physiology compensates this by increasing the SMA flow, and vice versa. The computational model takes this feature into account by increasing the corresponding flow in two cases:
- either when the initial 3D model has some geometrical feature that leads to a decreased CT or SMA blood flow as compared to typical, standard values (for example in cases of CT stenosis); or
- when the modification inputted in step c) leads to a decreased CT or SMA blood flow as compared to the simulated blood flows in step b).

The other fundamental mechanism implemented by the computational model is the hepatic artery buffer response (HABR). This regulatory mechanism allows to maintain a relatively constant value of hepatic blood flow by increasing the hepatic artery (and more specifically the PHA) blood flow in case of a decrease of the hepatic portal blood flow so that the total hepatic blood flow is restored. The computational model of the invention takes this mechanism into account by increasing, in a predefined amount, the modelled PHA blood flow if one of the modifications inputted in step c) corresponds to the ligation or clamping of the GDA and/or to the ligation or clamping of the SA, which are the main procedures during after which the portal vein system flow may decrease.

In order to fully characterize and test the accuracy of the proposed method, a tailored study was conducted including the analysis of 33 patients undergoing pancreaticoduodenectomy from January 2023 to September 2024, at a single institution. On the following, the overall process of implementation and validation of the proposed method will be discussed according to an embodiment of the present invention.

Patients' inclusion criteria were as follows: a) any indication for pancreaticoduodenectomy, b) Michels Type I arterial hepatic anatomy, c) absence of celiac trunk and superior mesenteric artery stenosis at preoperative CT scan, and d) informed consent. Exclusion criteria included: a) poor quality images, b) arterial abnormalities, such as infiltration of the arteries included in the segmentation and c) intraoperative inability to measure blood flow. Surgical procedure was performed and recorded for each patient, including open and robotic approaches. The CHA and PHA at the bifurcation of the GDA were selectively studied to create this computational model. Additionally, the developed model was tested in three new patients: two with radiologically significant CT stenosis and one patient with a replaced right hepatic artery (RHA) from SMA.

Patients' arterial phase images from MDCT in DICOM format were used to create the abdominal vascular system b3D models. Segmentation of the main arteries therein, including CHA, CT, GDA, and PHA, was done using an open-source platform for medical image analysis. Semi-automatic segmentation was applied to extract the targeted arteries' geometries (Figure 2, top left panel). After segmentation, the arterial geometries were reconstructed, and a mesh was generated using a computer-aided engineering software. Maximum mesh length was set at 0.3 mm for small arteries and 0.7 mm for the aorta to ensure high-quality meshes (Figure 2 top right panel).

Three meshes were created per case. Hemodynamic modelling was based on the two-parameter Windkessel (WK) model. WK parameters were tuned using patient-specific arterial flow measurements, whose obtention will be discussed in the following. Simulations were performed using a high-performance computational platform and included the simulation of the clamping of different arteries by setting zero flow at their outlet. For simplicity, constant flows were used instead of pulsatile flows, reducing computation time and model complexity with little to no detriment in the final results.

For all patients, intraoperative real-time blood flow measurement was obtained during PD with MiraQ^{™} Vascular device and Medistim Vascular TTFM Probes (MEDISTIM). Probe size was selected according to intraoperative size of CHA and GDA, generally 5 to 8 mm probes were used. All values were obtained in presence of hemodynamic stability and absence of vasoactive drugs and, in order to avoid physiological variability, blood flow was measured for 20 seconds at a time and the annotated value was the mean blood flow during these 20 seconds, including both respiratory phases. After dissecting hepatic hilum, transit-time ultrasound flowmeter was used (Figure 2, bottom left panel), and flow curves were recorded (Figure 2, bottom right panel). First, PHA, CHA and GDA flows were measured in baseline conditions. Subsequently, GDA was temporally clamped and PHA and CHA flows were measured (scenario 1). Finally, CHA was temporally clamped and PHA and GDA flows were measured (scenario 2). Subsequently, GDA was divided, and the surgical procedure was completed. Blood flow measurement variability could show an error margin between 10 to 15% according to the intraoperative conditions and probe position against the vessel, as defined by MEDISTIM.

Those measurements served as a reference for testing the method and were used to tune the computational model parameters' initial values. Another possibility is to use patient-specific variables to infer initial arterial flow without direct measurements. Within this latter setup, the predicted flows were validated through simulations in five patients. GDA and CHA clamping scenarios were simulated, comparing expected and actual PHA flows. Predictions were considered accurate when the real PHA flow fell within the predicted flow range.

To reduce computational complexity, the pancreaticoduodenal arcade was simplified by connecting the distal GDA and the IPDA. Resistances were adjusted accordingly in the Windkessel model. Within this setup, simulations were conducted for open, GDA-clamped, and CHA-clamped scenarios. The first 15 patients were used to calibrate the computational model, while patients 16 to 33 were used for validation. WK parameters were adjusted iteratively based on blood flow measurements. Aortic pressure was fixed at 100 mmHg, although adjusting it proportionally would not change flow distribution due to resistance adjustments. Simplified representations of pancreaticoduodenal arcades further reduced computational costs. The model was also tested in two patients with celiac trunk stenosis and one patient with a replaced right hepatic artery (Michel's Type 8), confirming its applicability to varied anatomical conditions.

Table 1 shows intraoperative collected patient data of patients 1 to 15. Intraoperative flow measurements were in line with the physical expected behavior of blood flow following the principle of continuity. Initial CHA flow decreases when GDA is clamped (scenario 1), whereas PHA flow increases.

These data from patients 1 to 15 were used to adjust the model. In patients without CT stenosis the simulation showed that the fluid followed the expected path, pumping blood from CHA to GDA and its arcade; Fig 3 (top left panel) shows the CFD model in the initial anatomical configuration. The model was considered to be well calibrated when the difference between the flow measured during surgery and the simulated flow was less than 1%, which is negligible compared to the error margin of the probes used, typically between 10% and 15%. All panels in Figure 3 show in different shades and tones the blood's velocity in cm/s. Scale is not detailed as this Figure is shown as a representative, qualitative example.

The selected WK parameters produced reliable and reproducible results. Simulated blood flow closely matched intraoperative measurements, demonstrating the model's accuracy. Simulated arterial wave pulses were comparable to real arterial waveforms recorded during surgery, including both systolic and diastolic peaks. Additionally, the simulations showed lower velocities near the arterial walls, consistent with physiological flow patterns observed in clinical settings.

| **ID** | **CHA (ml/min)** | **GDA (ml/min)** | **CHA1 (clamped GDA) (ml/min)** | **GDA1 (clamped CHA) (ml/min)** |
|---|---|---|---|---|
| **ID1** | 374 | -155 | 344 | 104 |
| **ID2** | 112 | -49 | 98 | 6 |
| **ID3** | 244 | -90 | 209 | 92 |
| **ID4** | 257 | -94 | 458 | 100 |
| **ID5** | 246 | -82 | 234 | 104 |
| **ID6** | 374 | -155 | 344 | 104 |
| **ID7** | 465 | -93 | 390 | 63 |
| **ID8** | 226 | -78 | 156 | 78 |
| **ID9** | 125 | -110 | 85 | 105 |
| **ID10** | 179 | -52 | 262 | 203 |
| **ID11** | 133 | -15 | 112 | 22 |
| **ID12** | 302 | -95 | 219 | 29 |
| **ID13** | 209 | -54 | 163 | 121 |
| **ID14** | 214 | -69 | 209 | 92 |
| **ID15** | 385 | -70 | 347 | 99 |

Table 1

All these results can be seen in Tables 2 and 3 for simulation scenarios 1 and 2, respectively. In scenario 1 (with a clamped GDA) the predicted PHA flow increases after GDA clamping, showing a relative error of less than 10%. This margin of error is likely due to inherent variability in flow measurements. The model demonstrated 100% accuracy, as real PHA flows after GDA clamping consistently fell within the expected ranges provided by the simulations. In all cases, measured PHA flow exceeded the predicted minimum, ensuring that the liver's inflow was not underestimated, thus reducing potential postoperative risks. Figure 3 (top right panel) illustrates the new arterial geometry after GDA clamping, with velocity patterns aligning closely with realistic clinical flow behavior.

In scenario 2 (with a clamped CHA), the anatomical complexity in the pancreatic head region and the non-hypertrophic pancreaticoduodenal arcade made segmentation challenging, increasing the computational cost. Despite these difficulties, the model produced accurate predictions. As seen in Table 3, the model achieved 80% accuracy in predicting PHA flow after CHA clamping, with errors mainly attributed to the mentioned segmentation challenges. Figure 3 (bottom left panel) depicts the new arterial geometry and flow pattern, while Figure 3 (bottom right panel) illustrates blood flow redistribution after CHA clamping. Within the bottom panels of Figure 3, the comparison between real, measured PHA blood flow and predicted PHA by using the method of the invention can be used as exemplary of the accuracy of said method. Left: comparison between intraoperative measured PHA blood flow and predicted PHA blood flow after GDA clamping (scenario 1) is 314 ml/min vs 247 - 331 ml/min, respectively; right: comparison between intraoperative measured PHA blood flow and predicted PHA blood flow after CHA clamping (scenario 2) is is 91 ml/min vs 89 - 118 ml/min, respectively.

| **ID** | **CHA (ml/min)** | **GDA (ml/min)** | **PHA₀* (ml/min)** | **PHA₁ real (ml/min)** | **Prediction PHA₁ (ml/min)** | **Prediction PHA_{1 min} (ml/min)** |
|---|---|---|---|---|---|---|
| **ID16** | 331 | 60 | 271 | 314 | 289,2 | 247,1 |
| **ID17** | 144 | 18 | 126 | 120 | 129,6 | 112,5 |
| **ID18** | 179 | 52 | 127 | 136 | 130,8 | 105,1 |
| **ID19** | 215 | 54 | 161 | 153 | 171,6 | 142,3 |
| **ID20** | 213 | 67 | 146 | 176 | 159,6 | 128,25 |
| **ID24** | 880 | 50 | 830 | 828 | 837,0 | 792,0 |
| **ID25** | 140 | 80 | 60 | 165 | 88,8 | 74,4 |
| **ID26** | 360 | 70 | 290 | 258 | 304,2 | 257,7 |
| **ID27** | 300 | 25 | 275 | 265 | 276,8 | 241,25 |
| **ID28** | 390 | 20 | 370 | 373 | 374,0 | 371,6 |
| **ID29** | 460 | 100 | 360 | 420 | 439,0 | 399,6 |
| **ID30** | 150 | 60 | 90 | 97 | 97.2 | 93.6 |
| **ID31** | 250 | 35 | 215 | 220 | 204,6 | 194,2 |
| **ID32** | 230 | 40 | 190 | 195 | 212,2 | 201,0 |
| **ID33** | 200 | 23 | 177 | 180 | 180,0 | 178,2 |

Table 2

In both Tables 2 and 3, ID corresponds to the patient identification number; CHA to the measured initial common hepatic artery flow; GDA to the measured initial gastroduodenal artery flow, PHA₀* to the inferred initial proper hepatic artery flow, and PHA₁/PHA₂ to the proper hepatic artery flow after GDA clamping. Errors are to be interpreted as calculated with the difference between predicted and real PHA₁ over real PHA. Relative error over the minimum expected value is calculated as (real PHA₁-min PHA₁)/real PHA₁. Table 3 errors are to be interpreted as are calculated with the difference between predicted and real PHA₂ over real PHA.

| **ID** | **CHA (ml/min)** | **PHA₀* (ml/min)** | **GDA (ml/min)** | **Real PHA₂ (ml/min)** | **Prediction AHP₂ (ml/min)** |
|---|---|---|---|---|---|
| **ID16** | 331 | 271 | 60 | 91 | 103,2 |
| **ID17** | 144 | 126 | 18 | 43 | 47,5 |
| **ID18** | 179 | 127 | 52 | 203 | 74,5 |
| **ID19** | 215 | 161 | 54 | 98 | 102,3 |
| **ID20** | 213 | 146 | 67 | 99 | 106,2 |
| **ID28** | 390 | 370 | 20 | 270 | 90,6 |
| **ID29** | 460 | 360 | 100 | 211 | 234,6 |
| **ID31** | 250 | 215 | 35 | 68 | 50,2 |
| **ID32** | 230 | 190 | 40 | 64 | 47,2 |
| **ID33** | 200 | 177 | 23 | 61 | 54,2 |

Table 3

### Anatomical Variations

The CFD model successfully simulated patients with anatomical variations. For instance, the blood flow simulation performed for one of the patients with a replaced right hepatic artery (RHA) originating from the SMA, demonstrated accurate flow redistribution predictions. After GDA clamping, the liver received blood primarily from the celiac trunk, with minimal redistribution in the accessory hepatic ducts (AHD). The predicted RHA blood flow was 59 ml/min, closely matching the intraoperative measurement of 67 ml/min (range: 56.95-77.05 ml/min), considering a 15% variability in the measurement device. Figure 4 highlights these flow patterns.

On the other hand, two patients with celiac trunk stenosis were also modeled using geometries extracted directly from MDCT scans. The resulting CFD models revealed distinct blood flow distributions compared to other patients, with the GDA becoming the primary source of liver perfusion rather than the CHA. Figure 5 illustrates blood flow redistribution in a patient with clinically significant celiac trunk stenosis, highlighting the unique hemodynamic adjustments caused by this condition.

Again, right panel in Figure 4 and middle and right panels in Figure 5 show in different shades and tones the blood's velocity in cm/s. Scale is not detailed as this Figures are shown as a representative, qualitative examples.

When analyzing the patient with a clinically significant celiac trunk stenosis, the 3D reconstruction showed a higher GDA section compared to non-pathological CT scans. By combining the higher GDA area with the reduced CT area, the model autonomously simulated an initial GDA blood flow directed to the liver. This reversal of the blood flow as shown by the computational model confirms that CFD is able to interpret the CT stenosis as an anatomical variation that affects the redistribution of the blood flow. It was not necessary to modify the model in order to obtain these results. Therefore, it can be assumed that the developed model is capable of simulating different conditions than those used for its development

These positive results demonstrate the effectiveness of the method of the invention in predicting blood flow redistribution after an anatomical modification is carried out in the abdominal vascular system of a subject. Thus, the method of the invention represents a viable and reliable methodology to predict hepatic arterial flow, among other relevant blood flows involved in HPB surgery. To achieve this, the current particular focus has been set on the CHA and the PHA located at the bifurcation of the GDA. The intraoperative data measured in the patients showed that the studied arterial system behaves as expected by the laws of fluid biophysics (Table 1). On one hand, the initial CHA flow decreases when GDA is clamped (scenario 1) because the total flow coming from the aorta is redistributed to the remaining open arterial branches of the entire abdominal aorta. Nevertheless, the flow in the PHA after GDA clamping increases compared to the initial PHA flow due to the flow redistribution. On the other hand, intraoperatively, the reversion and flow magnitude of the GDA after CHA clamping showed the relevance of analyzing the communication between these two arterial systems when assessing supramesocolic arterial vascularization.

The developed methodology has demonstrated reproducibility, and the current model has shown good accuracy in the different studied scenarios. The developed (computational) CFD model can predict the flow distribution in the study area in initial anatomy, scenario 1 and scenario 2, showing the minimum expected arterial blood flow.

However, physical laws are not able to fully explain the increase in PHA blood flow after GDA clamping, as, in all cases, the real increase in blood flow is higher than the predicted. This is related to the HABR, that, in case of a decrease of the hepatic portal blood flow, restores total hepatic perfusion by increasing the HA blood flow. Accordingly, once the GDA is clamped, blood flow distributed through the pancreaticoduodenal arcade decreases, reducing the blood that is subsequently drained through the portal vein system. This decrease in hepatic portal vein blood flow can activate the HABR, increasing CHA and/or PHA blood flow. This increase is added in the proposed method to the expected blood flow redistribution explained by physical laws, as commented earlier. Based on this argument, by adjusting the parameters in this scenario, the model can be adapted to the expected biological behavior, showing a reproducible blood flow behavior in all cases.

The developed method, and particularly the computational model comprised therein, effectively predicts blood flow variations in the CHA and PHA at the GDA bifurcation (among other abdominal vessels), a critical anatomical landmark in major liver and pancreatic surgeries.

## Claims

1. Computer-implemented method for predicting post-surgical blood flow redistribution in a subject's abdominal vascular system, said method comprising performing the following steps:
a) providing a three-dimensional, 3D, digital model of a vascular system of an abdominal region of the subject, based on one or more medical images of said abdominal region;
b) generating an initial computational model of blood flow through the subject's abdominal vascular system, based on the 3D digital model;
c) receiving input information indicating one or more blood-flow modification conditions at a location of the subject's abdominal vascular system;
d) updating the computational model to include the blood-flow modification conditions inputted in step c); and
e) outputting a representation of the redistribution of the abdominal blood flow of the subject after the abdominal vascular system modification/s inputted in step c) according to the model updated in step d);
**characterized in that:**
- the 3D model built in step a) comprises the computational segmentation of at least the common hepatic artery, CHA, the gastroduodenal artery, GDA, the celiac trunk, CT, and the CT arcades, the proper hepatic artery, PHA, the superior mesenteric artery, SMA, the splenic artery, SA, and the inferior pancreaticoduodenal artery, IPDA, within the one or more medical images of the abdominal region of the subject, and the posterior reconstruction of said vessels' geometries;
- the computational model is adapted to apply a biological mechanism of blood flow compensation between the CT and the SMA, by increasing, in a predefined amount, the modelled SMA blood flow if the 3D model provided in step a) or if one of the modifications inputted in step c) is related to a reduction in the normal CT blood flow;
- the computational model is adapted to apply the biological mechanism of blood flow compensation between the CT and the SMA, by increasing, in a predefined amount, the modelled CT blood flow if the 3D model provided in step a) or if one of the modifications inputted in step c) is related to a reduction in the normal SMA blood flow;
- the computational model is adapted to apply a biological mechanism of blood flow compensation between the CT and the SMA, by increasing, in a predefined amount, the modelled CT/SMA blood flow if the 3D model provided in step a) or if one of the modifications inputted in step c) is related to a reduction in the normal SMA/CT blood flow; and
- the computational model updated in step d) is adapted to apply the hepatic artery buffer response, HABR, by increasing, in a predefined amount, the modelled PHA blood flow if one of the modifications inputted in step c) corresponds to the ligation or clamping of the GDA and/or to the ligation or clamping of the SA.

2. Method according to claim 1, wherein the computational model obtained in step b) and updated in step d) comprises an algorithm based on computational fluid dynamics.

3. Method according to claim 2, wherein the algorithm comprises a Windkessel, WK, model.

4. Method according to any one of claims 2-3, wherein the algorithm is based on artificial intelligence, AI, techniques adapted to provide test data of the blood flow through a test subject's abdominal vascular system based on a 3D digital model of said vascular system using as input real or synthetic physiological training data of a plurality of training subjects.

5. Method according to any one of claims 2-4, wherein the algorithm comprises a set of parameters and wherein the parameter's initial values are set using:
- data associated with intraoperative measurements of arterial and/or venous blood flows of the subject; and/or
- data inferred by means of executing an AI algorithm adapted to provide an estimate of an initial blood flow of one or more abdominal vessels using as input at least one of the following: data from blood flow intraoperative measurements of a plurality of subjects, subject's demographics, subject's clinical variables, and subject's imaging-derived features.

6. Method according to claim 5 wherein the data associated with intraoperative measurements and/or the data inferred by means of executing an AI algorithm comprise data relating to the abdominal blood flow during HABR of:
- one or more subjects with a healthy liver; and/or
- one or more subjects with hepatic cirrhosis requiring a liver transplant.

7. Method according to any one of claims 3-6, wherein the WK model comprises fixed parameter values except for a resistance parameter.

8. Method according to any one of claims 1-7, wherein the one or more medical images of the abdominal region of said subject comprise a computed tomography scan and/or a multidetector computer tomography scan.

9. Method according to any one of claims 1-8, wherein at least one of the modifications inputted in step c) comprises one of the following: clamping or ligation of the CHA, clamping or ligation of the GDA and clamping or ligation of the SA.

10. Method according to any one of claims 1-9, wherein:
- steps c)-e) are repeated iteratively;
- the information inputted each time that step c) is carried out is based on a blood flow modification condition of the subject occurring in real time; and
- the update of the model and the output of the representation of the redistribution of the abdominal blood flow of the subject according to each modification condition inputted in step c) are carried out in real time.

11. Apparatus for predicting post-surgical blood flow redistribution in a subject's abdominal vascular system, said apparatus comprising computing means comprising hardware and/or software means adapted to perform a method according to any one of claims 1-10.

12. Apparatus according to claim 11 further comprising medical image acquisition means adapted to obtain one or more images of the abdominal region of a subject.

13. Apparatus for predicting post-surgical blood flow redistribution in a subject's abdominal vascular system comprising:
- computing means comprising hardware and/or software means adapted to perform a method according to any one of claims 5-10; and
- one or more medical probes adapted to measure intraoperatively the blood flow in a vessel.

14. Apparatus according to claim 13, further comprising medical image acquisition means adapted to obtain one or more images of the abdominal region of a subject.

15. Computer program comprising instructions which, when the program is executed by computing means, cause the computing means to carry out a method according to any one of claims 1-10.
